# EUROPEAN PATENT APPLICATION

(11) **EP 3 610 775 A1**
(43) Date of publication of application: **19.02.2020**
(21) Application number: 19192053.7
(22) Date of filing: 16.08.2019
(51) Int. Cl.: A61B 1/00, A61B 1/018, A61B 5/06, A61B 1/05, A61B 17/00

(54) **ENDOSCOPE WITH ANATOMY ELEVATION ASSEMBLY**

(30) Priority: 17.08.2018 US 201862765168 P; 17.07.2019 US 201916513959
(71) Applicant: Acclarent, Inc., Irvine, CA 92618 (US); Biosense Webster (Israel) Ltd., Yokneam, 2066717 (IL)
(72) Inventor: PALUSHI, Jetmir, Irvine California 92618 (US); AKBARIAN, Fatemeh, Irvine California 92618 (US); FANG, Itzhak, Irvine California 92618 (US); PAPADAKIS, Athanasios, Irvine California 92618 (US); SHAMELI, Ehsan, Irvine California 92618 (US); ALGAWI, Yehuda, Yokneam 2066717 (IL); GOVARI, Assaf, Yokneam 2066717 (IL); DEAN, Marc, Fort Worth Texas 76104 (US); EBRAHIMI, Babak, Irvine California 92618 (US); TROTT, Jordan R., Irvine California 92618 (US)
(74) Representative: Small, Gary James

(57) **Abstract**

An endoscope assembly includes a shaft assembly, an endoscopic camera assembly, and an anatomy elevation assembly. The shaft assembly includes a flexible outer shaft defining a lumen, a first electrical communication line, and a first fluid communication line. The endoscopic camera assembly is in communication with the first electrical communication line. The anatomy elevation assembly includes an inflatable member coupled to the flexible outer shaft. The inflatable member includes an interior surface. The first fluid communication line is in fluid communication with the inflatable member. The inflatable member is configured to transition between a deflated configuration and an inflated configuration. The interior surface is configured to define a viewing path distal to the endoscopic camera assembly while the inflatable member is in the inflated configuration.

## Description

### PRIORITY

This application claims priority to U.S. Provisional Pat. App. No. 62/765,168, entitled "Endoscope with Anatomy Elevation Assembly," filed August 17, 2018, the disclosure of which is incorporated by reference herein.

### BACKGROUND

Various endoscopes may be used to provide a line of sight for targeted anatomy during biopsies and other surgical procedures. For example, a variable direction view endoscope may be used to provide visualization within an anatomical passageway (e.g., the ear, nose, throat, paranasal sinuses, etc.) to diagnose or operate surrounding tissue in the area. A variable direction view endoscope may enable viewing along a variety of transverse viewing angles without having to flex the shaft of the endoscope within the anatomical passageway. Such an endoscope that may be provided in accordance with the teachings of U.S. Pub. No. 2010/0030031, entitled "Swing Prism Endoscope," published February 4, 2010, the disclosure of which is incorporated by reference herein.

Image-guided surgery (IGS) is a technique where a computer is used to obtain a real-time correlation of the location of an instrument that has been inserted into a patient's body to a set of preoperatively obtained images (e.g., a CT or MRI scan, 3-D map, etc.), such that the computer system may superimpose the current location of the instrument on the preoperatively obtained images. In some IGS procedures, a digital tomographic scan (e.g., CT or MRI, 3-D map, etc.) of the operative field is obtained prior to surgery. A specially programmed computer is then used to convert the digital tomographic scan data into a digital map. During surgery, special instruments having sensors (e.g., electromagnetic coils that emit electromagnetic fields and/or are responsive to externally generated electromagnetic fields) mounted thereon are used to perform the procedure while the sensors send data to the computer indicating the current position of each surgical instrument. The computer correlates the data it receives from the instrument-mounted sensors with the digital map that was created from the preoperative tomographic scan. The tomographic scan images are displayed on a video monitor along with an indicator (e.g., crosshairs or an illuminated dot, etc.) showing the real-time position of each surgical instrument relative to the anatomical structures shown in the scan images. In this manner, the surgeon is able to know the precise position of each sensor-equipped instrument by viewing the video monitor even if the surgeon is unable to directly visualize the instrument itself at its current location within the body.

An example of an electromagnetic IGS systems that may be used in ENT and sinus surgery is the CARTO® 3 System by Biosense-Webster, Inc., of Irvine, California. When applied to functional endoscopic sinus surgery (FESS), balloon sinuplasty, and/or other ENT procedures, the use of IGS systems allows the surgeon to achieve more precise movement and positioning of the surgical instruments than can be achieved by viewing through an endoscope alone. As a result, IGS systems may be particularly useful during performance of medical procedures where anatomical landmarks are not present or are difficult to visualize endoscopically.

While several systems and methods have been made and used in medical procedures, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1 depicts a perspective view of an exemplary sinus surgery instrument having an exemplary guide shaft assembly with a flexible portion;
FIG. 2 depicts a schematic view of an exemplary sinus surgery navigation system being used on a patient seated in an exemplary medical procedure chair;
FIG. 3A depicts an elevational side view of an exemplary endoscope assembly that maybe readily incorporated with the sinus surgery instrument of FIG. 1, where an anatomy elevation assembly is in a deflated configuration;
FIG. 3B depicts an elevational side view of the endoscope assembly of FIG. 3A, where the anatomy elevation assembly is in an inflated configuration;

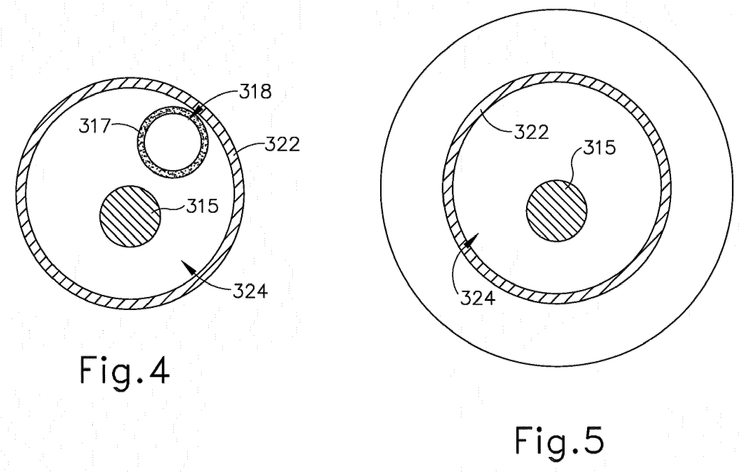

FIG. 4 depicts a cross-sectional view of the endoscope assembly of FIG. 3A, taken along line 4-4 of FIG. 3A;
FIG. 5 depicts a cross-sectional view of the endoscope assembly of FIG. 3A, taken along line 5-5 of FIG. 3A;
FIG. 6A depicts a cross-sectional side view of the flexible portion of the guide shaft assembly of FIG. 1 and the endoscope assembly of FIG. 3A, where the guide shaft assembly is inserted within a patient adjacent to an anatomical passage, where the distal end of the endoscope assembly is housed within the flexible portion of the guide shaft assembly while the anatomy elevation assembly is in the deflated configuration;
FIG. 6B depicts a cross-sectional side view of the flexible portion of the guide shaft assembly of FIG. 1 and the endoscope assembly of FIG. 3A, where the guide shaft assembly is inserted within a patient adjacent to an anatomical passage, where the distal end of the endoscope assembly is distal relative to the flexible portion of the guide shaft assembly while the anatomy elevation assembly is in the deflated configuration;
FIG. 6C depicts a cross-sectional side view of the flexible portion of the guide shaft assembly of FIG. 1 and the endoscope assembly of FIG. 3A, where the guide shaft assembly is inserted within a patient adjacent to an anatomical passage, where the distal end of the endoscope assembly is distal relative to the flexible portion of the guide shaft assembly while the anatomy elevation assembly is in the inflated configuration;
FIG. 7 depicts a cross-sectional view of the flexible portion of the guide shaft assembly of FIG. 1 and the endoscope assembly of FIG. 3A, taken along line 7-7 of FIG. 6A;
FIG. 8A depicts a cross-sectional side view of the distal end of an alternative exemplary endoscope assembly that may be readily incorporated with the sinus surgery instrument of FIG. 1 and the sinus surgery navigation system of FIG. 2, where an anatomy elevation assembly is in a deflated configuration;
FIG. 8B depicts cross-sectional side view of the distal end of the endoscope assembly of FIG. 8A, where the anatomy elevation assembly is in an inflated configuration;

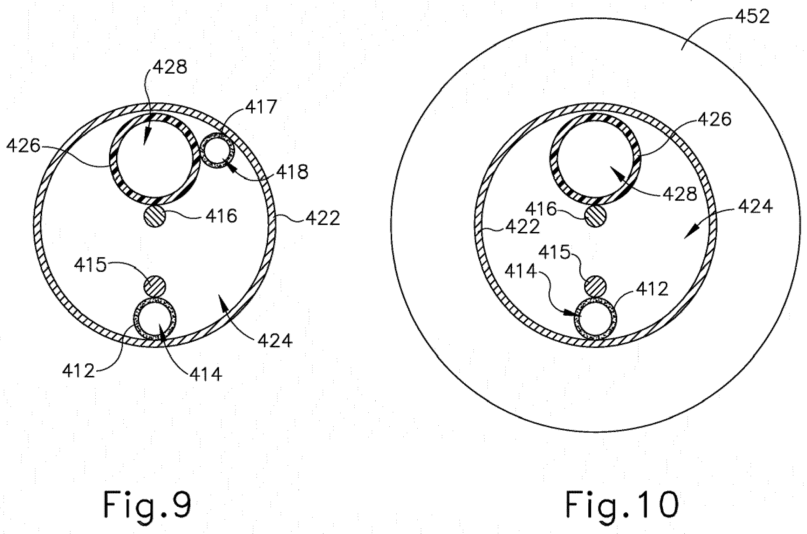

FIG. 9 depicts a cross-sectional view of the endoscope assembly of FIG. 8A, taken along line 9-9 of FIG. 8A;
FIG. 10 depicts a cross-sectional view of the endoscope assembly of FIG. 8A, taken along line 10-10 of FIG. 8A;
FIG. 11A depicts a cross-sectional side view of the flexible portion of the guide shaft assembly of FIG. 1 and the endoscope assembly of FIG. 8A, where the guide shaft assembly is inserted within a patient adjacent to an anatomical passage, where the distal end of the endoscope assembly is housed within the flexible portion of the guide shaft assembly while the anatomy elevation assembly is in the deflated configuration;
FIG. 11B depicts a cross-sectional side view of the flexible portion of the guide shaft assembly of FIG. 1 and the endoscope assembly of FIG. 8A, where the guide shaft assembly is inserted within a patient adjacent to an anatomical passage, where the distal end of the endoscope assembly is distal relative to the flexible portion of the guide shaft assembly while the anatomy elevation assembly is in the deflated configuration;
FIG. 11C depicts a cross-sectional side view of the flexible portion of the guide shaft assembly of FIG. 1 and the endoscope assembly of FIG. 8A, where the guide shaft assembly is inserted within a patient adjacent to an anatomical passage, where the distal end of the endoscope assembly is distal relative to the flexible portion of the guide shaft assembly while the anatomy elevation assembly is in the inflated configuration;
FIG. 11D depicts a cross-sectional side view of the flexible portion of the guide shaft assembly of FIG. 1 and the endoscope assembly of FIG. 8A, where the guide shaft assembly is inserted within a patient adjacent to an anatomical passage, where the distal end of the endoscope assembly is distal relative to the flexible portion of the guide shaft assembly while the anatomy elevation assembly is in the inflated configuration, where a flexible forceps instrument extends distally from the endoscope assembly;
FIG. 12 depicts a cross-sectional side view of the flexible portion of the guide shaft assembly of FIG. 1 and the endoscope assembly of FIG. 8A, where the guide shaft assembly is inserted within a patient adjacent to an anatomical passage, where the distal end of the endoscope assembly is distal relative to the flexible portion of the guide shaft assembly while the anatomy elevation assembly is in the inflated configuration, where a flexible swab instrument extends distally from the endoscope assembly; and
FIG. 13 depicts a cross-sectional side view of the flexible portion of the guide shaft assembly of FIG. 1 and the endoscope assembly of FIG. 8A, where the guide shaft assembly is inserted within a patient adjacent to an anatomical passage, where the distal end of the endoscope assembly is distal relative to the flexible portion of the guide shaft assembly while the anatomy elevation assembly is in the inflated configuration, where a spray tube instrument extends distally from the endoscope assembly.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

It is further understood that any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

### I. Overview of Exemplary Instrument

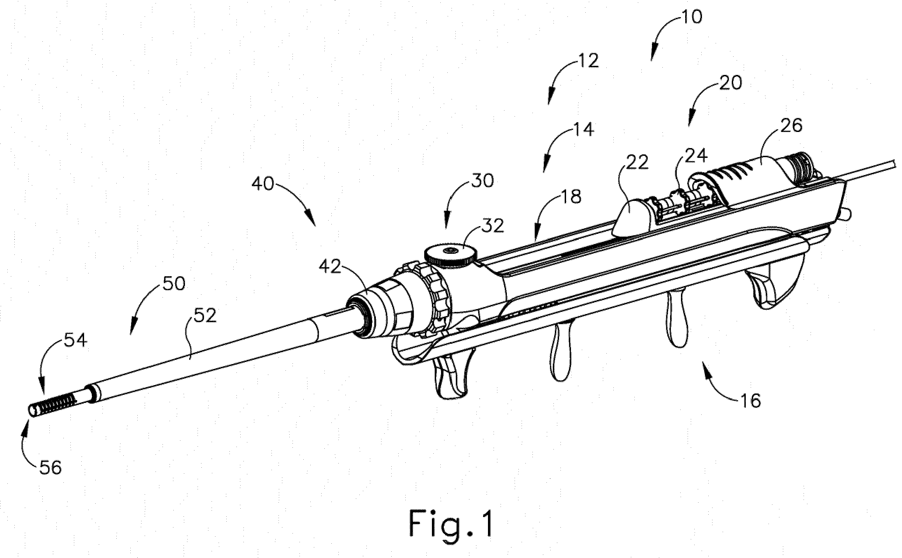

FIG. 1 shows an exemplary instrument (10) that may be used to provide access within or adjacent to various different anatomical passageways (e.g., frontal sinus ostium, frontal recess, maxillary sinus ostium, sphenoid sinus ostium, ethmoid sinus ostium, Eustachian tube, etc.). Instrument (10) includes a handle assembly (12), an actuating assembly (20), a rotation drive assembly (30), an articulation drive assembly (40), and a guide shaft assembly (50). As will be described in greater detail below, handle assembly (12) and actuating assembly (20) may be configured to allow an operator to control the placement of guide shaft assembly (50), as well as various working elements disposed in guide shaft assembly (50), with a single hand. Additionally, as will be described in greater detail below, rotation drive assembly (30) and articulation drive assembly (40) may be used to provide access to various different anatomical passageway with the same guide shaft assembly (50) by making simple adjustments to structural features of instrument (10).

Handle assembly (12) includes a body portion (14) and a grip portion (16). Actuating assembly (20) includes a first actuator (22) and a second actuator (26). Body portion (14) and grip portion (16) may be detachable from each other such that different suitable grip portions (16) may couple with different suitable body portions (14). Actuators (22, 26) may be suitably coupled with at least one working element for each actuator (22, 26). For instance, actuators (22, 26) may couple with a respective guide wire, the shaft of a balloon catheter, the shaft of a flexible endoscope, the shaft of a biopsy flexible forceps, or any other working element that would be apparent to one skilled in the art in view of the teaching herein. In the present example, each actuator (22, 26) is operable to translate along body portion (14) independently of the other actuator (22, 26).

The operator may slide either actuator (22, 26) along body portion (14) with the same hand grasping handle assembly (12) such that a working element may relative to handle assembly (12) and guide shaft assembly (50); and further such that a distal portion of a corresponding working element extends distally past open distal end (56) of guide shaft assembly (50). Thus, when open distal end (56) of guide shaft assembly (50) is placed within a desired location, the operator may translate actuator (22, 26) such that a corresponding working element extends past open distal end (56) toward the desired anatomical structure. In the current example, second actuator (26) defines a cavity that first actuator (22) may translate through such that second actuator (26) does not inhibit translation of first actuator (22) and vice versa. First actuator (22) includes a rotating body (24) that is configured to rotate about its own longitudinal axis. Rotating body (24) may couple with a working element such that rotation of rotating body (24) about its own longitudinal axis drives rotation of the associated working element about its own longitudinal axis. In some variations, one actuator (22, 26) may be omitted.

Guide shaft assembly (50) includes a rigid guide shaft portion (52) and a flexible guide shaft portion (54) terminating at open distal end (56). Guide shaft (50) may be dimensioned to be inserted transnasally or transorally to provide access to suitable anatomical structures within a patient's head via open distal end (56). In other exemplary uses, guide shaft (50) is positioned elsewhere within a patient (e.g., somewhere other than within the patient's head). Various suitable locations and procedures in which instrument (10) may be used will be apparent to those skilled in the art in view of the teachings herein.

Rigid guide shaft portion (52) is coupled with a thumbwheel (32) of rotation drive assembly (30). By way of example only, rigid guide shaft portion (52) and thumbwheel (32) may be coupled together via complementary bevel gears. Such a coupling may be provided in accordance with at least some of the teachings of U.S. Pat. App. No. 16/032,471, entitled "Adjustable Instrument for Dilation of Anatomical Passageway," filed July 11, 2018, the disclosure of which is incorporated by reference herein. Other suitable ways in which rigid guide shaft portion (52) and thumbwheel (32) may be coupled together will be apparent to those skilled in the art in view of the teachings herein. In the present example, thumbwheel (32) of rotation drive assembly (30) is operable to rotate guide shaft assembly (50), relative to handle assembly (12), about the longitudinal axis of guide shaft assembly (50). Thumbwheel (32) is rotatable about an axis that is perpendicular to the longitudinal axis of guide shaft assembly (50) in this example. Other suitable features that may be used to provide rotation of guide shaft assembly (50) will be apparent to those skilled in the art in view of the teachings herein. Alternatively, instrument (10) may be configured such that guide shaft assembly (50) is not rotatable relative to handle assembly (12).

Articulation drive assembly (40) is configured to selectively drive articulation of flexible guide shaft portion (54) relative to the longitudinal axis defined by guide shaft assembly (50). Articulation drive assembly (40) is operable to cause flexible guide shaft portion (54) to flex, to thereby deflect open distal end (56) away from the longitudinal axis of rigid guide shaft portion (52). Articulation drive assembly (40) includes a knob (42) that is coaxially disposed about rigid guide shaft portion (52). Knob (42) is rotatable, relative to rigid guide shaft portion (52), about the longitudinal axis of rigid guide shaft portion (52). Knob (42) is coupled with distal end (56) of flexible guide shaft portion (54) such that rotation of knob (42) about rigid guide shaft portion (52) causes deflection of distal end (56). In some versions, knob (42) is coupled with distal end (56) of flexible guide shaft portion (54) via a push-pull cable. By way of further example only, articulation drive assembly (40) and flexible guide shaft portion (54) may be configured and operable in accordance with at least some of the teachings of U.S. Pat. App. No. 16/032,471, entitled "Adjustable Instrument for Dilation of Anatomical Passageway," filed July 11, 2018, the disclosure of which is incorporated by reference herein; and/or U.S. Pat. App. No. 15/955,232, entitled "Deflectable Guide for Medical Instrument," filed April 17, 2018; and U.S. Pat. App. No. 62/555,841, entitled "Adjustable Instrument for Dilation of Anatomical Passageway," filed September 8, 2017, the disclosure of which is incorporated by reference herein.

### II. Exemplary Image Guided Surgery Navigation System

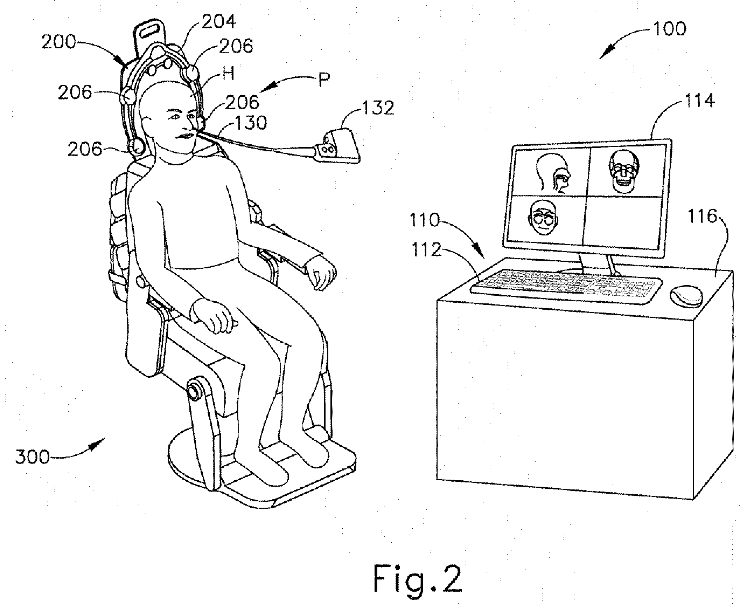

FIG. 2 shows an exemplary IGS navigation system (100) enabling a medical procedure to be performed using image guidance. In addition to or in lieu of having the components and operability described herein IGS navigation system (100) may be constructed and operable in accordance with at least some of the teachings of U.S. Pat. Pub. No. 2014/0364725, entitled "Systems and Methods for Performing Image Guided Procedures within the Ear, Nose, Throat and Paranasal Sinuses," published December 11, 2014, the disclosure of which is incorporated by reference herein; and/or U.S. Patent App. No. 15/933,737, entitled "Apparatus to Secure Field Generating Device to Chair," filed March 23, 2018, the disclosure of which is incorporated by reference herein.

IGS navigation system (100) of the present example comprises a field generator assembly (200), which comprises set of magnetic field generators (206) that are integrated into a horseshoe-shaped frame (204). Field generators (206) are operable to generate alternating magnetic fields of different frequencies around the head of the patient. Field generators (206) thereby enable tracking of the position of a navigation guidewire (130) that is inserted into the head of the patient. In the present example, frame (204) is mounted to a chair (300), with the patient (P) being seated in the chair (300) such that frame (204) is located adjacent to the head (H) of the patient (P).

IGS navigation system (100) of the present example further comprises a processor (110), which controls field generators (206) and other elements of IGS navigation system (100). For instance, processor (110) is operable to drive field generators (206) to generate electromagnetic fields; and process signals from a navigation guidewire (130) to determine the location of a sensor (not shown) in navigation guidewire (130) within the head (H) of the patient (P). Processor (110) of the present example is mounted in a console (116), which comprises operating controls (112) that include a keypad and/or a pointing device such as a mouse or trackball. A coupling unit (132) is secured to the proximal end of a navigation guidewire (130). Coupling unit (132) may provide wired or wireless communication of data and other signals between console (116) and navigation guidewire (130).

Navigation guidewire (130) of the present example includes a sensor (not shown) that is responsive to the alternating electromagnetic fields generated by field generators (206). In the present example, the sensor of navigation guidewire (130) comprises at least one wire coil at the distal end of navigation guidewire (130). When such a coil is positioned within an alternating electromagnetic field generated by field generators (206), that the alternating electromagnetic field may generate electrical current in the coil, and this electrical current may be communicated along the electrical conduit(s) in navigation guidewire (130) and further to processor (110) via coupling unit (132). This phenomenon may enable IGS navigation system (100) to determine the location of the distal end of navigation guidewire (130) within a three-dimensional space (i.e., within the head (H) of the patient (P)). To accomplish this, processor (110) executes an algorithm to calculate location coordinates of the distal end of navigation guidewire (130) from the position related signals of the coil(s) in navigation guidewire (130).

Processor (110) is further operable to provide video in real time via display screen (114), showing the position of the distal end of navigation guidewire (130) in relation to a video camera image of the patient's head (H), a CT scan image of the patient's head (H), and/or a computer generated three-dimensional model of the anatomy within and adjacent to the patient's nasal cavity. Display screen (114) may display such images simultaneously and/or superimposed on each other during the surgical procedure. Such displayed images may also include graphical representations of instruments that are inserted in the patient's head (H), such as navigation guidewire (130), such that the operator may view the virtual rendering of the instrument at its actual location in real time. By way of example only, display screen (114) may provide images in accordance with at least some of the teachings of U.S. Pub. No. 2016/0008083, entitled "Guidewire Navigation for Sinuplasty," published January 14, 2016, the disclosure of which is incorporated by reference herein. In the event that the operator is also using an endoscope, the endoscopic image may also be provided on display screen (114).

### III. Exemplary Endoscope with Anatomy Elevation Assembly

In certain areas within a patient's anatomy (e.g., within the ear, nose, or throat), it may be difficult to obtain a satisfactory field of view with a conventional endoscope. These limitations may be due in part to complicated anatomical passages being difficult to access. Additionally, flexible endoscopes having an articulating distal end may have too large a profile to access certain targeted anatomical structures (e.g., a Eustachian tube or sinus cavity, etc.). In some instances, it may be difficult to obtain endoscopic visualization of a complicated anatomical passage without having to dissect tissue. For example, when using an endoscope with a rigid shaft, dissecting tissue may be required to provide a suitable amount of clearance to accommodate for the rigid shaft. In other instances, collapsed tissue may block the lens of an endoscope from suitably viewing surrounding tissue in the area.

Therefore, it may be desirable to have a low profile flexible endoscope that may access complicated anatomical passages. It may also be desirable to use a low profile flexible endoscope in conjunction with a guide shaft, like guide shaft assembly (50), to accurately place the low profile flexible endoscope within an anatomical passageway. It may further be desirable to have an endoscope capable of elevating anatomy away from the lens of an endoscope to view surrounding tissue in the area. Additionally, it may be desirable to have an endoscope with a working channel capable of accessing the surrounding tissue with additional instrumentation while the endoscope elevates anatomy away from the lens.

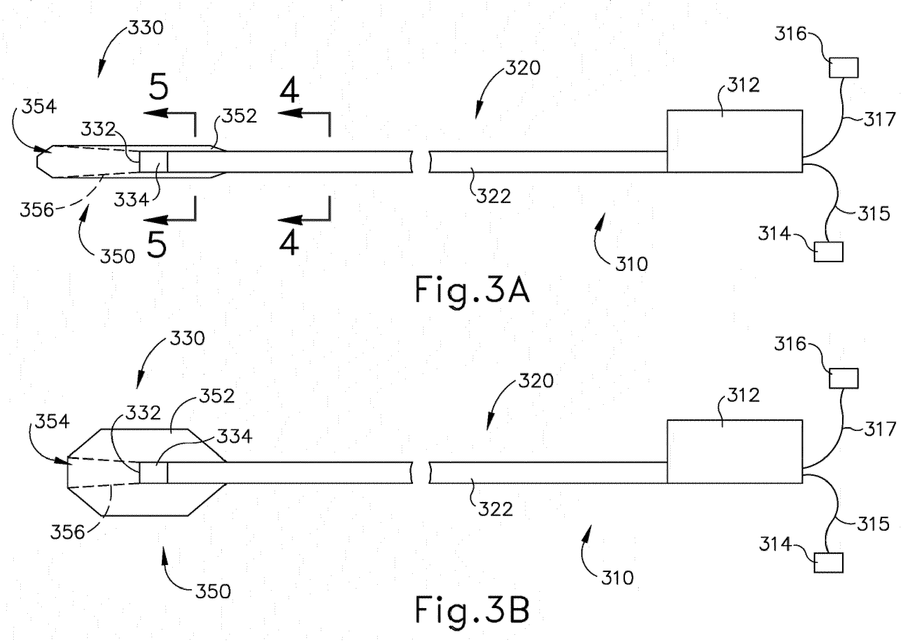

FIGS. 3A-3B show an exemplary endoscope assembly (310) that may be used to access complicated anatomical passages, such as those that may be encountered during medical procedures within the ear, nose, or throat (or elsewhere). Endoscope assembly (310) may be readily incorporated into instrument (10) described above. Endoscope assembly (310) of the current example includes an actuator (312), a shaft assembly (320), an endoscopic camera assembly (330), and an anatomy elevation assembly (350). As will be described in greater detail below, shaft assembly (320), endoscopic camera assembly (330), and anatomy elevation assembly (350) are dimensioned small enough to be slidably inserted within guide shaft (50) to suitably access complicated anatomical passages. As will also be described in greater detail below, anatomy elevation assembly (350) is configured to selectively engage and move tissue obstructing the view of endoscopic camera assembly (330) such that tissue is no longer an obstruction to endoscopic camera assembly. Actuator (312) may be substantially like either actuator (22, 26) described above. Actuator (312) may thus be configured to slidably couple with handle assembly (12) such that the operator may translate actuator (312) with the same hand that grasps handle assembly (12), to thereby translate shaft assembly (320) relative to handle assembly (12).

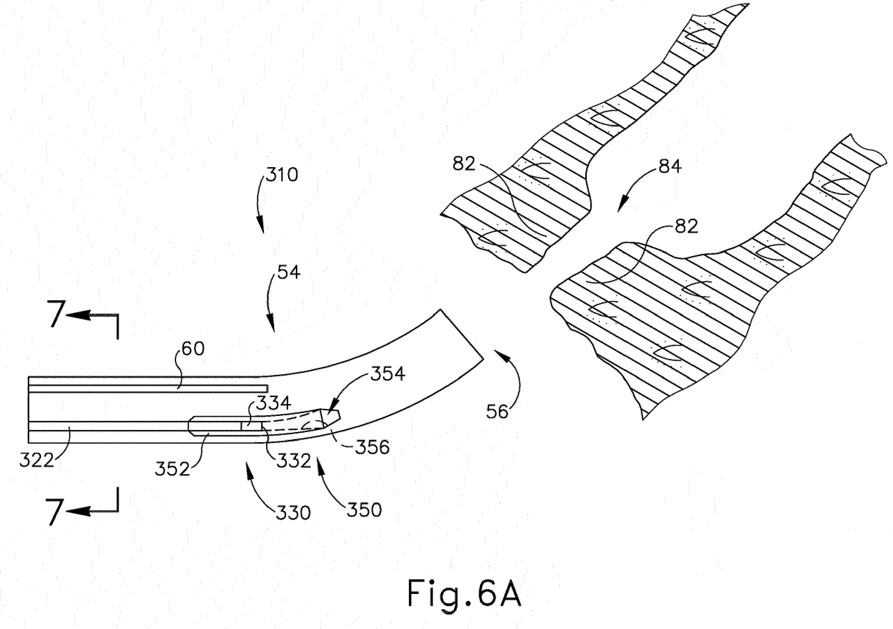

Shaft assembly (320) includes a flexible outer shaft (322) defining an interior lumen (324), an electrical connection line (315), and a fluid communication line (317). Flexible outer shaft (322) extends distally from actuator (312). In some instances, actuator (312) may include a rotating body substantially like rotating body (24) described above. In such instances, actuator (312) may be configured to rotate shaft assembly (320), as well as components attached to shaft assembly, about the longitudinal axis defined by shaft assembly (320). Flexible outer shaft (322) is dimensioned to slidably fit within guide shaft assembly (50) when actuator (312) is suitably coupled to handle assembly (12) of instrument (10). A distal end of flexible outer shaft (322) is attached to endoscopic camera assembly (330) and anatomy elevation assembly (350). As best seen in FIGS. 6A-6B, a distal portion of flexible outer shaft (322), endoscopic camera assembly (330), and anatomy elevation assembly (350) are dimensioned to fit within the interior of flexible guide shaft portion (54) of guide shaft assembly (50) when anatomy elevation assembly (350) is in a non-expanded configuration.

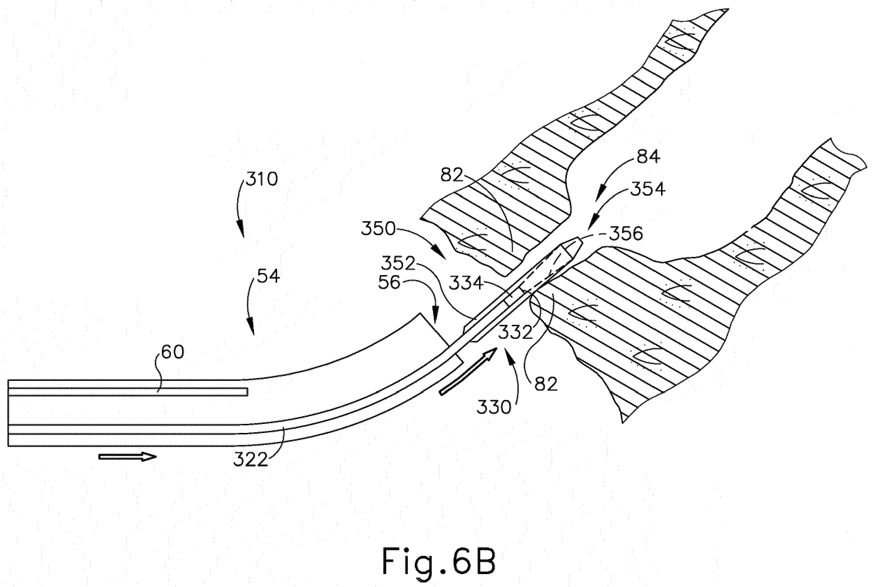

Flexible outer shaft (322) is operable to flex from a straight configuration (as shown in FIGS. 3A-3B and 6A) into a bent configuration (as shown in FIGS. 6B-6C) in response to an external force. Flexible outer shaft (322) may flex from the straight configuration into the bent configuration in response to being advanced distally through a bent flexible guide shaft portion (54) (as shown in FIGS. 6B-6C). Components housed within corresponding portions of flexible outer shaft (322) may bend along with flexible outer shaft (322). Flexible outer shaft (322) may also be substantially resilient such that when an external force is no longer present, flexible outer shaft (322) returns to the straight configuration.

Electrical communication line (315) provides a line of communication between first coupling feature (314) and endoscopic camera assembly (330). Electrical communication line (315) extends distally within interior lumen (324) into endoscopic camera assembly (330). Electrical communication line (315) also extends proximally from actuator (312) into first coupling feature (314). First coupling feature (314) is operable to connect with suitable components to activate endoscopic camera assembly (330) and communicate images captured via endoscopic camera assembly (330) in accordance with the description herein.

Fluid communication line (317) defines a fluid lumen (318). Fluid communication line (317) provides fluid communication between second coupling feature (316) and the interior of a balloon (352) of anatomy elevation assembly (350). Fluid communication line (317) extends distally within interior lumen (324) of flexible outer shaft (322) and terminates into the interior of balloon (352). Fluid communication line (317) also extends proximally from actuator (312) into second coupling feature (316). Second coupling feature (316) is operable to connect to components capable of suitably inflating and deflating balloon (352) in accordance with the description herein. By way of example only, second coupling feature (316) may couple with a syringe or other source of inflation fluid (e.g., saline, etc.).

As mentioned above, endoscopic camera assembly (330) is in communication with electrical communication line (315) and is attached to the distal end of flexible shaft (322). Endoscopic camera assembly (330) includes a camera portion (332) and a light portion (334). Camera portion (332) is configured to capture video images and transfer those video images to a video screen via electrical communication line (315) and first coupling feature (314). Light portion (334) is configured to provide a source of light, illuminating the field of view of camera portion (332), so that images captured by camera portion (332) are viewable. In the current example, camera portion (332) does not include driven articulation features, which may allow camera portion (332) and the distal end of shaft assembly (320) to have a relatively small profile (i.e., smaller than the profile of a conventional articulating endoscope) that is suitable for entrance within guide shaft assembly (50). This smaller profile may be around or smaller than 2 millimeters. However, this is merely optional, as some versions may include a camera portion (332) that has driven articulation features.

As mentioned above, anatomy elevation assembly (350) is configured to engage and move tissue obstructing the view of camera portion (332). Anatomy elevation assembly (350) includes balloon (352) having a proximal portion attached to the exterior of flexible outer shaft (322). Balloon (352) may be compliant, non-compliant, semi-compliant, etc. Any suitable balloon (352) may be utilized as would be apparent to one skilled in the art in view of the teachings herein. As mentioned above, the interior of balloon (352) is in fluid communication with fluid communication line (317) of shaft assembly (320). Fluid communication line (317) extends from interior lumen (324) to an exterior surface of flexible outer shaft (322) located within the confines of balloon (352). Of course, fluid communication line (317) may be in fluid communication with balloon (352) through any suitable means as would be apparent to one skilled in the art in view of the teachings herein.

The operator may selectively inflate balloon (352) by coupling a fluid source (e.g. a syringe) with second coupling feature (316) and driving fluid through fluid lumen (318) into the interior of balloon (352). When the operator wishes to move obstructing tissue away from camera portion (332), the operator may inflate balloon (352) such that the exterior surface of balloon (352) may engage and push away obstructing tissue. Alternatively, the operator may selective deflate balloon (352) by suctioning fluid from balloon (352) toward second coupling feature (316); or may otherwise relieve fluid pressure from balloon (352) to allow the fluid to drain toward second coupling feature (316).

While a proximal portion of balloon (352) is attached to the exterior of flexible outer shaft (322), a distal portion of balloon (352) includes an interior surface (356) extending distally from camera portion (332) in a frusto-conical fashion to define a frusto-conical viewing pathway (354). Viewing pathway (354) terminates distally into an open distal end such that material forming balloon (352) does not obstruct viewing pathway (354) of camera portion (332). Therefore, if balloon (352) happens to accumulate any fluids or other material, the accumulated fluid or material will not obstruct the view of camera portion (332). While in the current example, interior surface (356) extends distally from camera portion (332) in a frusto-conical fashion, any other suitable geometry may be used to define viewing pathway (354) as would be apparent to one skilled in the art in view of the teachings herein. For instance, interior surface (356) may extend outwardly in an arched fashion. As another merely illustrative example, viewing pathway (354) may be omitted, and the distal portion of balloon (352) may be disposed over camera portion (332). In such versions, balloon (352) may be transparent to enable camera portion (332) to see through balloon (352). Also in such versions, balloon (352) may include a hydrophobic material to reduce buildup of fluids, etc. on the exterior of balloon (352).

While anatomy elevation assembly (350) is incorporated into endoscope assembly (310) of the current example, this is merely optional. In some instances, anatomy elevation assembly (350) may be entirely omitted from endoscope assembly (310).

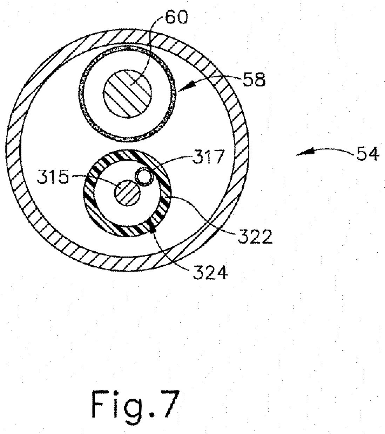

FIGS. 6A-7 show an exemplary use of endoscope assembly (310) in conjunction with instrument (10) to view an anatomical passageway (84) obstructed by collapsed tissue (82). As best seen in FIG. 7, guide shaft assembly (50) defines a working lumen (58) slidably housing a working element (60). As mentioned above, working element (60) may be attached to actuator (22, 26) and may include a guidewire, a flexible forceps instrument, or any other suitable working element as would be apparent to one skilled in the art in view of the teachings herein. The operator may actuate working element (60) independently of endoscope assembly (310). In the current example, working element (60) is slidably disposed in working lumen (58) defined by flexible guide shaft portion (54), such that working element (60) is laterally displaced from endoscope assembly (310). However, this is merely optional. For instance, working element (60) may be slidably housed within interior lumen (234) of flexible outer shaft (322).

First, as shown in FIG. 6A, the operator may insert guide shaft assembly (50) such that open distal end (56) is adjacent to the desired anatomical passageway (84). This positioning may include actuation of articulation drive assembly (40) to controllably deform flexible guide shaft portion (54), to thereby orient open distal end (56) along an axis that is oblique to the longitudinal axis of rigid guide shaft portion (52). With open distal end (56) placed in the desired location and orientation, the operator may advance actuator (312) in accordance with the description herein, such that endoscopic camera assembly (330) translates distally past open distal end (56).

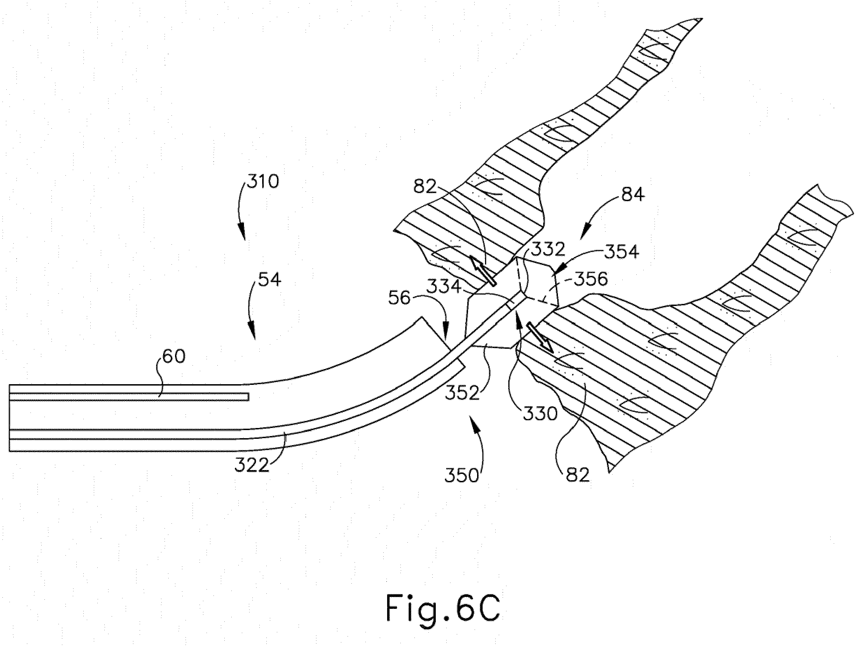

In some instances, as shown in FIG. 6B, collapsed tissue (82) or other anatomy may block the line of sight of endoscopic camera assembly (330) such that the operator may not be able to view all desired locations. In such an instance, as shown in FIG. 6C, the operatory may inflate balloon (352) of anatomy elevation assembly (350) in accordance with the description herein such that an exterior surface of balloon (352) displaces collapsed tissue (82) to provide a suitable field of view for endoscope camera assembly (330).

With balloon (352) inflated as shown in FIG. 6C, interior surface (356) expands into the frusto-conical shape such that viewing path (354) is defined. The open distal end the interior surface (356) of balloon (352) ensures that material comprising balloon (352) does not obstruct viewing path (354) either. At the stage shown in FIG. 6C, the operator may suitably view anatomical pathway (84) without collapsed tissue (82) obstructing the view of endoscopic camera assembly (330). When the operator desires to retract endoscope assembly (310), the operator may deflate balloon (352) in accordance with the description herein, and proximally translate actuator (312) until both endoscopic camera assembly (330) and anatomy elevation assembly (350) are within the confines of flexible guide shaft portion (54). Then the operator may remove flexible guide shaft portion (54) from the patient. At any suitable point during the procedure shown in FIGS. 6A-6C, the operator may also advance working element (60) distally through open distal end (56) and thereby use working element (60) in any suitable fashion.

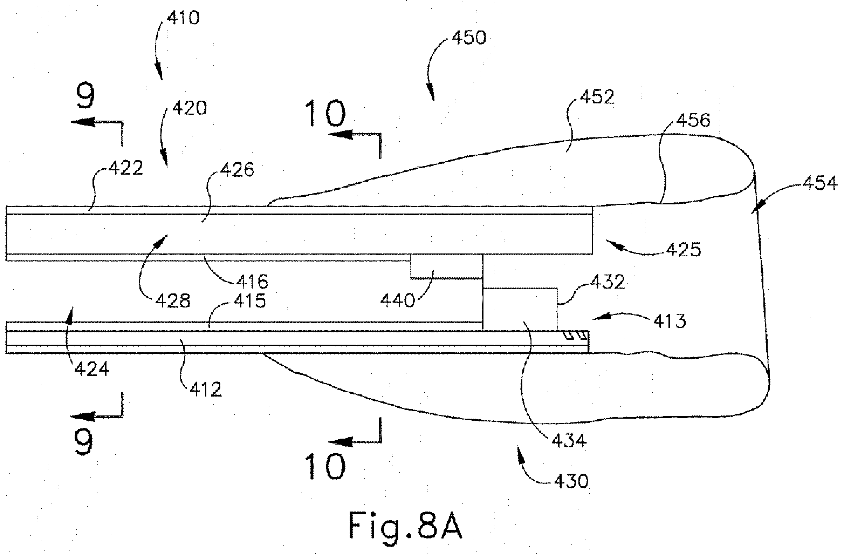

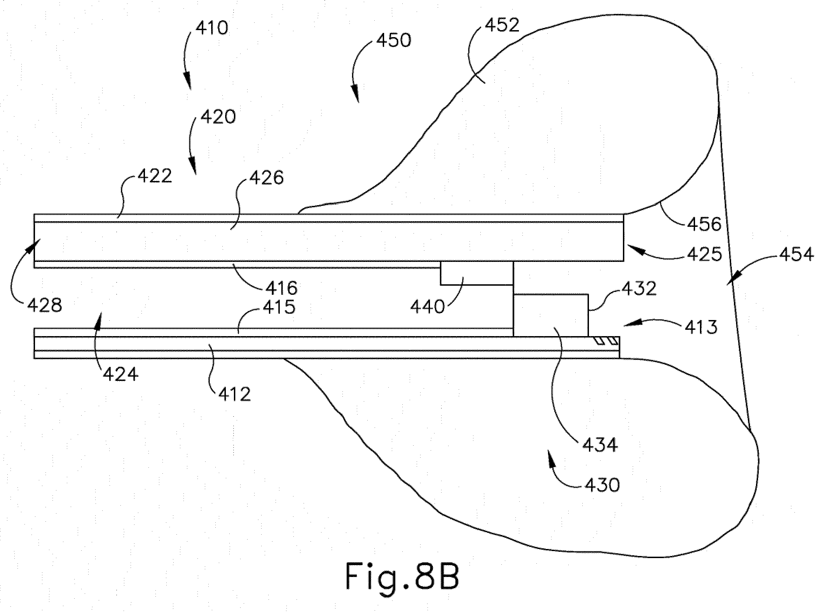

FIGS. 8A-8B show another exemplary endoscope assembly (410) that may be used to access complicated anatomical passages, such as those that may be encountered during medical procedures within the ear, nose, or throat (or elsewhere). Endoscope assembly (410) of this example includes a shaft assembly (420), an endoscopic camera assembly (430), an IGS sensor element (440), and an anatomy elevation assembly (450). Like endoscope assembly (310) described above, shaft assembly (420), endoscopic camera assembly (430), and anatomy elevation assembly (450) are dimensioned small enough to be slidably inserted within guide shaft (50) to suitably access complicated anatomical passages. Also like endoscope assembly (310) described above, anatomy elevation assembly (450) is configured to selectively engage and move tissue obstructing the view of endoscopic camera assembly (430) such that tissue is no longer an obstruction to endoscopic camera assembly (430).

As will be described in greater detail below, shaft assembly (420) is dimensioned to slidably receive a working element that may be suitably used while anatomy elevation assembly (450) is inflated. As will also be described in greater detail below, IGS navigation sensor (440) is configured to couple with IGS navigation system (100) such that the position of sensor (440) within the patient is viewable in real time via display screen (114) in relation to a video camera image of the patient's head (H) (or other anatomical structure), a CT scan image of the patient's head (H) (or other anatomical structure), and/or a computer generated three-dimensional model of the anatomy within and adjacent to the patient's nasal cavity (or other anatomical structure).

While not shown, endoscope assembly (410) includes an actuator that may be substantially like either actuator (22, 26, 312) described above. Such an actuator may be configured to slidably couple with handle assembly (12) such that the operator may translate the actuator with the same hand that grasps handle assembly (12), to thereby translate endoscope assembly (410) relative to handle assembly (12).

Shaft assembly (420) includes a flexible outer shaft (422) defining an interior lumen (424), a working catheter (426) defining a working lumen (428), a first electrical connection line (415), a second electrical connection line (416), a first fluid communication line (417), and a second fluid communication line (412). Flexible outer shaft (422) may extend distally from an actuator (not shown), which may be movably coupled with handle assembly (12) as noted above. In some instances, the actuator may include a rotating body substantially like rotating body (24) described above. In such instances, the actuator may be configured to rotate shaft assembly (420), as well as components attached to shaft assembly, about the longitudinal axis defined by shaft assembly (420).

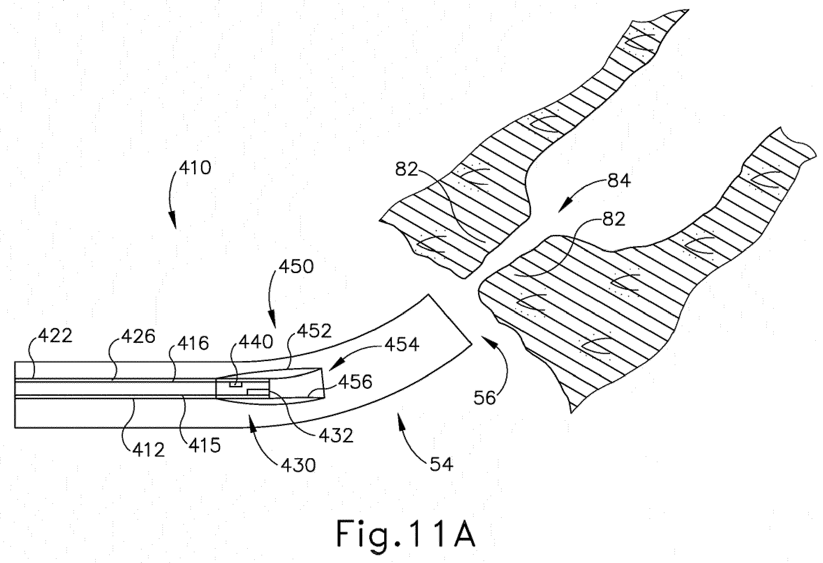

Flexible outer shaft (422) is dimensioned to slidably fit within guide shaft assembly (50) when the actuator is suitably coupled to handle assembly (12) of instrument (10). A distal end of flexible outer shaft (422) is attached to endoscopic camera assembly (430), IGS sensor element (440), and anatomy elevation assembly (450). Endoscopic camera assembly (430) and IGS sensor element (440) are housed within interior lumen (424) of flexible outer shaft (422). Flexible outer shaft (422) terminates distally at an open distal end (425) such that the line of sight for endoscopic camera assembly (430) is not obstructed by distal end (425) of flexible outer shaft (422); and also such that a working element may extend past the distal end (425) of flexible outer shaft (422) and working catheter (426) during exemplary use. As best seen in FIGS. 11A-11B, a distal portion of flexible outer shaft (422), endoscopic camera assembly (430), IGS sensor element (440), and anatomy elevation assembly (450) are dimensioned to fit within the interior of flexible guide shaft portion (54) of guide shaft assembly (50) when anatomy elevation assembly (450) is in a non-expanded configuration.

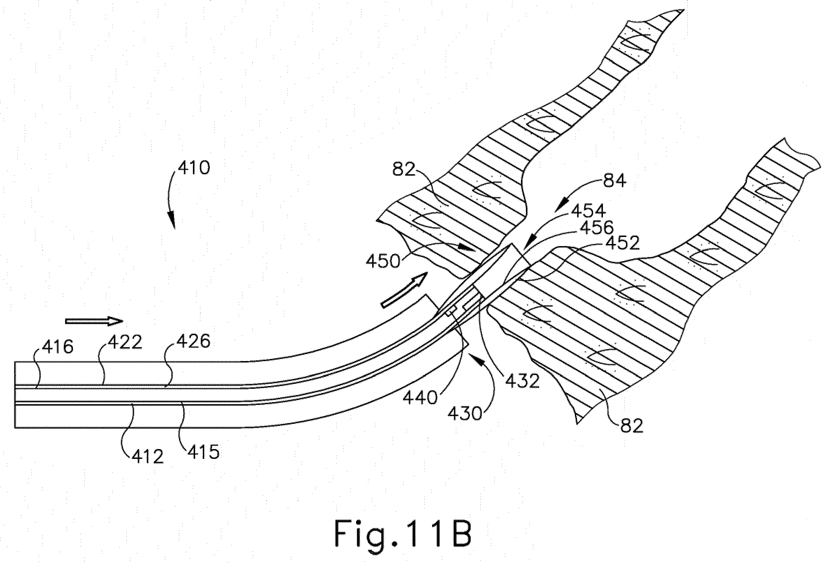

Flexible outer shaft (422) is operable to flex from a straight configuration (as shown in FIGS. 3A-3B and 11A) into a bent configuration (as shown in FIGS. 11B-11D) in response to an external force. Flexible outer shaft (422) may flex from the straight configuration into the bent configuration in response to being advanced distally through a bent flexible guide shaft portion (54) (as shown in FIGS. 11B- 11D). Components housed within corresponding portions of flexible outer shaft (422) may bend along with flexible outer shaft (422). Flexible outer shaft (422) may also be substantially resilient such that when an external force is no longer present, flexible outer shaft (422) returns to the straight configuration.

Working catheter (426) is housed within interior lumen (424) of flexible outer shaft (422). Working lumen (428) of working catheter (426) is dimensioned to slidably receive a working element during exemplary use of anatomy elevation feature (450). When anatomy elevation feature (450) is activated to achieve the expanded configuration, the operator may advance the working element distally through working lumen (428) past an open distal end (425) of flexible outer shaft (422).

First electrical communication line (415) provides a line of communication between a first coupling feature (not shown) and endoscopic camera assembly (430). Like electrical communication line (315), first electrical communication line (415) extends distally within interior lumen (424) into endoscopic camera assembly (430). First electrical communication line (415) also extends proximally from the actuator into a first coupling feature. This first coupling feature may be substantially like first coupling feature (314) described above such that the first coupling feature is operable to connect with suitable components to activate endoscopic camera assembly (430) and communicate images captured via endoscopic camera assembly (430) in accordance with the description herein.

Second electrical communication line (416) provides a line of communication between a coupling (not shown) unit of IGS navigation system (100) and IGS sensor element (440). Second electrical communication line (416) extends distally within interior lumen (343) into IGS sensor element (440). Second electrical communication line (416) also extends proximally from actuator into the coupling unit of IGS navigation system (100). This coupling unit may be substantially like coupling unit (132) described above such that IGS sensor element (440) is in communication with console (116) via the coupling unit.

First fluid communication line (417) defines a fluid lumen (418). First fluid communication line (417) provides fluid communication between second coupling feature (416) and the interior of a balloon (452) of anatomy elevation assembly (450). First fluid communication line (417) extends distally within interior lumen (424) of flexible outer shaft (422) and terminates into the interior of balloon (452). First fluid communication line (417) also extends proximally from the actuator into a second coupling feature (not shown). This second coupling feature is substantially like second coupling feature (316) described below such that the second coupling feature is operable to connect to components capable of suitably inflating and deflating balloon (452) in accordance with the description herein. By way of example only, this second coupling feature may couple with a syringe or other source of inflation fluid (e.g., saline, etc.).

Second fluid communication line (414) defines a fluid lumen (412). Second fluid communication line (414) provides fluid communication between a fluid coupling feature and fluid ports (413). Second fluid communication line (414) extends distally within interior lumen (424) of flexible outer shaft (422) and terminates into fluid ports (413). Second fluid communication line (414) also extends proximally from the actuators into another fluid couple feature (not shown) that is configured to fluidly couple with an irrigation fluid source. Fluid ports (413) are positioned and dimensioned to spray fluid traveling from irrigation source, through second fluid communication line (414) and onto the lens of camera assembly (430) to clean off undesirable residue on camera assembly (430) in order to provide a clear line of sight during exemplary use. In some versions, second fluid communication line (414) and fluid ports (413) are omitted.

IGS navigation sensor (440) is responsive to the alternating electromagnetic fields generated by field generators (206). In the present example, the sensor (440) comprises at least one wire coil at distal end (425) of flexible outer shaft (422). When such a coil is positioned within an alternating electromagnetic field generated by field generators (206), the alternating electromagnetic field may generate electrical current in the coil, and this electrical current may be communicated along second electrical communication wire (416) and further to processor (110) via coupling unit. This phenomenon may enable IGS navigation system (100) to determine the location of the distal end of exemplary endoscope assembly (410) within a three-dimensional space (e.g., within the head (H) of the patient (P)). To accomplish this, processor (110) executes an algorithm to calculate location coordinates of the distal end of endoscope assembly (410) from the position related signals of sensor (440). In some versions, sensor (440) and associated components are omitted.

As mentioned above, endoscopic camera assembly (430) is in communication with electrical communication line (415) and is attached to a distal portion of interior lumen (424) of flexible shaft (422). Endoscopic camera assembly (430) includes a camera portion (432) and a light portion (434). Camera portion (432) is configured to capture video images and transfer those video images to a video screen via electrical communication line (415) and first coupling feature (414). Light portion (434) is configured to provide a source of light, illuminating the field of view of camera portion (432), so that images captured by camera portion (432) are viewable. In the current example, camera portion (432) does not include driven articulation features, which may allow camera portion (432) and the distal end of shaft assembly (420) to have a relatively small profile (i.e., smaller than the profile of a conventional articulating endoscope) that is suitable for entrance within guide shaft assembly (50). This smaller profile may be around or smaller than 2 millimeters. However, this is merely optional, as some versions may include a camera portion (432) that has driven articulation features.

As mentioned above, anatomy elevation assembly (450) is configured to engage and move tissue obstructing the view of camera portion (432). Anatomy elevation assembly (340) includes balloon (452) having a proximal portion attached to the exterior of flexible outer shaft (422). Balloon (452) may be compliant, non-compliant, semi-compliant, etc. Any suitable balloon (452) may be utilized as would be apparent to one skilled in the art in view of the teachings herein. As mentioned above, the interior of balloon (452) is in fluid communication with fluid communication line (417) of shaft assembly (420). Fluid communication line (417) extends from interior lumen (424) to an exterior surface of flexible outer shaft (422) located within the confines of balloon (452). Of course, fluid communication line (417) may be in fluid communication with balloon (452) through any suitable means as would be apparent to one skilled in the art in view of the teachings herein.

The operator may selectively inflate balloon (452) by coupling a fluid source (e.g. a syringe) with a fluid coupling feature (not shown) and driving fluid through fluid lumen (418) into the interior of balloon (452). When the operator wishes to move obstructing tissue away from camera portion (432), the operator may inflate balloon (452) such that the exterior surface of balloon (452) may engage and push away obstructing tissue. Alternatively, the operator may selective deflate balloon (452) by suctioning fluid from balloon (452) toward second coupling feature (416); or may otherwise relieve fluid pressure from balloon (452) to allow the fluid to drain toward second coupling feature (416).

While a proximal portion of balloon (452) is attached to the exterior of flexible outer shaft (422), a distal portion of balloon (452) includes an interior surface (456) extending distally from camera portion (432) in a laterally outward expanding, arched fashion in order to define a viewing pathway (454). Viewing pathway (454) terminates distally into an open distal end such that material forming balloon (452) does not obstruct viewing pathway (454) of camera portion (432). Therefore, if balloon (452) happens to accumulate any fluids or other material, the accumulated fluid or material will not obstruct the view of camera portion (432). Additionally, viewing pathway (454) provides a path for a working element to extends distally past open distal end (425) while balloon (452) is inflated. While in the current example, interior surface (456) extends distally from camera portion (432) in a laterally expanding, arched fashion, any other suitable geometry may be used to define viewing pathway (454) as would be apparent to one skilled in the art in view of the teachings herein. For instance, interior surface (456) may extend outwardly in a frusto-conical fashion.

FIGS. 11A-11D show an exemplary use of endoscope assembly (410) in conjunction with instrument (10) to view an anatomical passageway (84) obstructed by collapsed tissue (82). In the current example, a working element in the form of forceps instrument (460) is slidably disposed in working catheter (426). While forceps instrument (460) is used in the current example, any other suitable working element may be used as would be apparent to one skilled in the art in view of the teachings herein.

First, as shown in FIG. 11A, the operator may insert guide shaft assembly (50) such that open distal end (56) is adjacent to the desired anatomical passageway (84). This positioning may include actuation of articulation drive assembly (40) to controllably deform flexible guide shaft portion (54), to thereby orient open distal end (56) along an axis that is oblique to the longitudinal axis of rigid guide shaft portion (52). As shown in FIG. 11B, with open distal end (56) placed in the desired location and orientation, the operator may actuate an actuator in accordance with the description herein such that open distal end (425) of flexible outer shaft (422) translates distally past open distal end (56), such that endoscopic camera assembly (430) actuates past open distal end (56). If IGS navigation system (100) is being, IGS navigation system (100) may track IGS sensor element (440) and display the position of IGS sensor element (440) and/or endoscope assembly (410) on display screen (114) in real time.

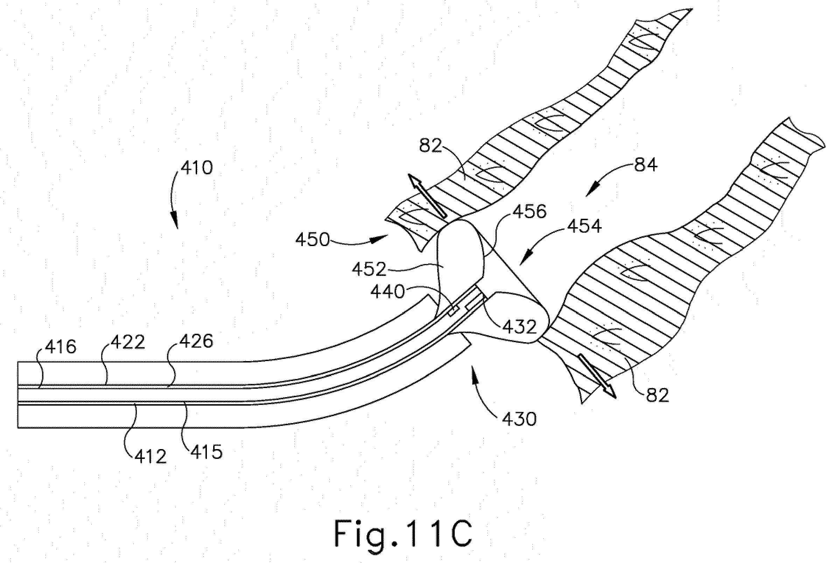

In some instances, as shown in FIG. 11B, collapsed tissue (82) or other anatomy may block the line of sight of endoscopic camera assembly (430) such that the operator may not being able to view all desired locations. In such an instance, as shown in FIG. 11C, the operatory may inflate balloon (452) of anatomy elevation assembly (450) in accordance with the description herein such that an exterior surface of balloon (452) displaces collapsed tissue (82) to provide a suitable field of view for endoscope camera assembly (430).

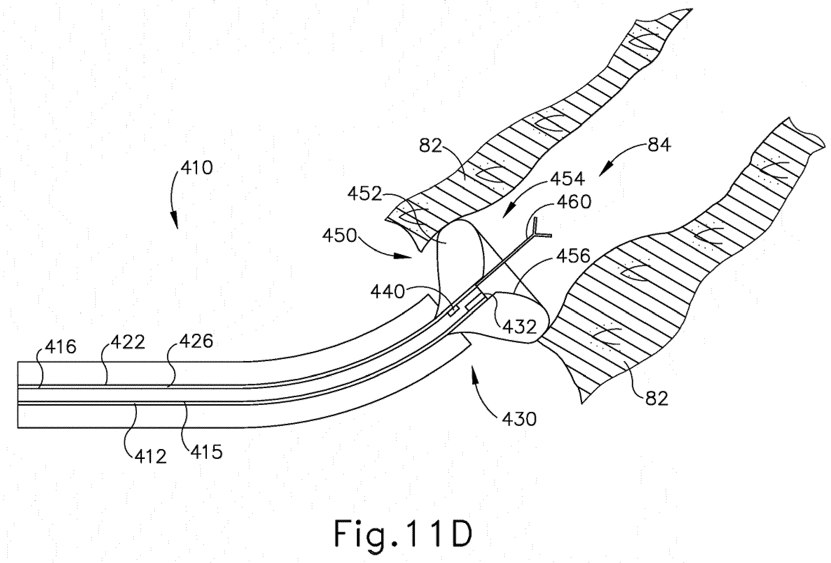

With balloon (452) inflated as shown in FIG. 11C, interior surface (456) expands into the laterally expanding, arched fashion such that viewing path (454) is defined. The open distal end the interior surface (456) of balloon (452) ensures that material comprising balloon (452) does not obstruct viewing path (454) either. At the stage shown in FIG. 11C, the operator may suitably view anatomical pathway (84) without collapsed tissue (82) obstructing the view of endoscopic camera assembly (430). As shown in FIG. 11D, the operator may also advance forceps instrument (460) distally through viewing path (454) and distally past balloon (452), to interact with anatomy as would be apparent to one skilled in the art in view of the teachings herein.

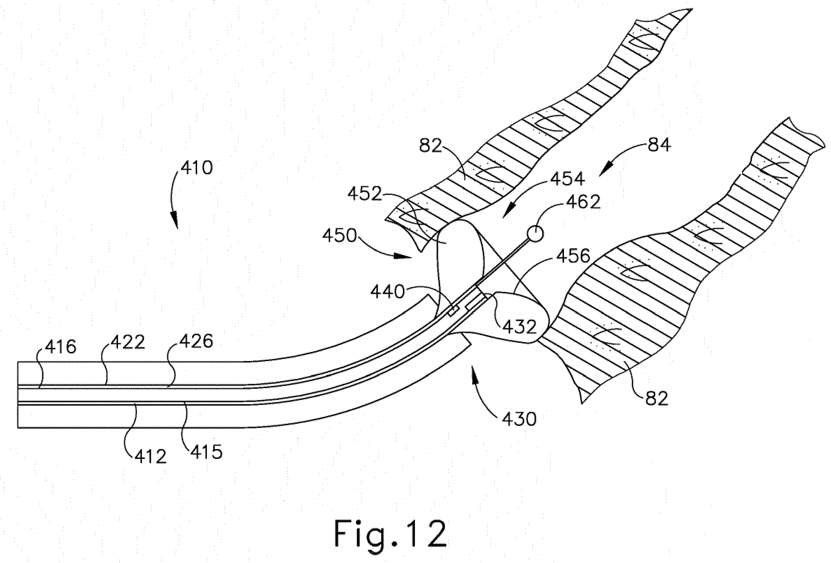

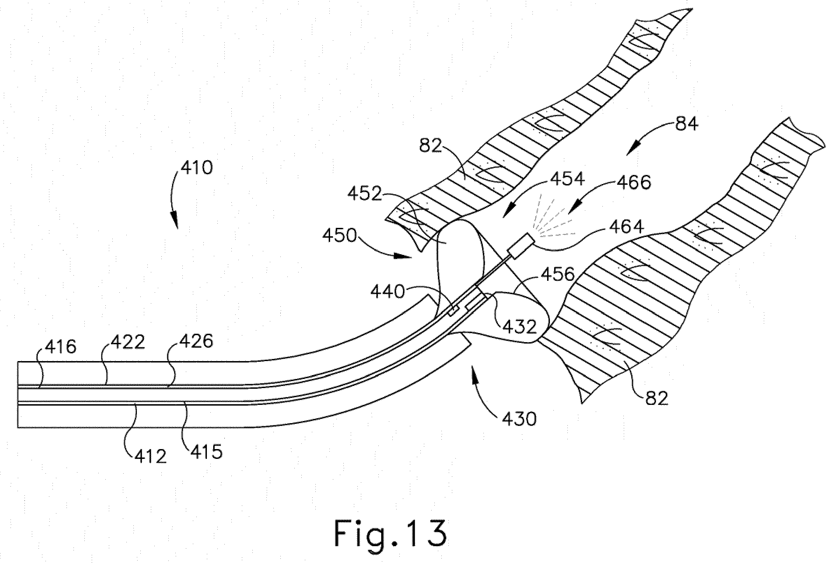

As mentioned above, while forceps instrument (460) is used in the current example, any other suitable working element may be used as would be apparent to one skilled in the art in view of the teachings herein. For example, as shown in FIG. 12, instead of advancing forceps instrument (460), the operator may advance a flexible swab (462) within and distally past working catheter (426) in order to take a diagnostic sample prior to treatment of the accessed anatomy. In some instances, the flexible swab (462) may be able to reach anatomical areas that are out of the line of sight. In some instances, the flexible swab (462) may be preloaded within the confines of working catheter (426) such that a distal end of flexible swab (462) is initially housed proximally relative to the distal end of working catheter (426). Additionally, as shown in FIG. 13, a spray tube (466) may be used as the working element in order to apply a suitable treatment fluid (466), or a variety of treatment fluids (466) to the accessed anatomy. In some instances, one working element may be used in accordance with the description herein, removed, and replaced with a second working element. The second working element may be used in accordance with the description herein and removed. Additionally, a third working element may be used, removed, replaced, etc.

When the operator desires to retract endoscope assembly (410), the operator may deflate balloon (452) in accordance with the description herein, and proximally translate the actuator until both endoscopic camera assembly (430) and anatomy elevation assembly (450) are within the confines of flexible guide shaft portion (54). Then the operator may remove flexible guide shaft portion (54) from the patient.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An endoscope assembly, comprising: (a) a shaft assembly, wherein the shaft assembly comprises: (i) a flexible outer shaft defining a lumen, (ii) a first electrical communication line, and (iii) a first fluid communication line; (b) an endoscopic camera assembly associated with the shaft assembly, wherein the endoscopic camera assembly is in communication with the first electrical communication line; and (c) an anatomy elevation assembly comprising an inflatable member coupled to the flexible outer shaft, wherein the inflatable member comprises an interior surface, wherein the first fluid communication line is in fluid communication with the inflatable member, wherein the inflatable member is configured to transition between a deflated configuration and an inflated configuration, wherein the interior surface is configured to define a viewing path distal to the endoscopic camera assembly while the inflatable member is in the inflated configuration.

### Example 2

The endoscope assembly of Example 1, wherein the fluid communication line extends at least partially within the lumen.

### Example 3

The endoscope assembly of any one or more of Examples 1 through 2, wherein the electrical communication line extends at least partially within the lumen.

### Example 4

The endoscope assembly of any one or more of Examples 1 through 3, wherein the interior surface is configured to define a frusto-conical fashion while the inflatable member is in the inflated configuration.

### Example 5

The endoscope assembly of any one or more of Examples 1 through 3, wherein the interior surface is configured to extend in an arched fashion while the inflatable member is in the inflated configuration.

### Example 6

The endoscope assembly of any one or more of Examples 1 through 5, wherein the shaft assembly further comprises a working catheter extending within the lumen of the flexible outer shaft, wherein the working catheter is dimensioned to slidably receive a working element.

### Example 7

The endoscope assembly of any one or more of Examples 1 through 6, wherein the inflatable member comprises a proximal portion and a distal portion, wherein the proximal portion is attached to the flexible outer shaft, wherein the distal portion includes the interior surface.

### Example 8

The endoscope assembly of any one or more of Examples 1 through 7, further comprising a second electrical communication line in communication with a sensor element housed within the lumen.

### Example 9

The endoscope assembly of Example 8, wherein the sensor element is operable to generate a position-indicative signal in response to an alternating magnetic field.

### Example 10

The endoscope assembly of any one or more of Examples 8 through 9, wherein the sensor element comprises a coil.

### Example 11

The endoscope assembly of any one or more of Examples 1 through 10, further comprising a second fluid communication line in communication with a fluid port configured to spray fluid on the camera assembly.

### Example 12

The endoscope assembly of any one or more of Examples 1 through 11, further comprising an actuator attached to a proximal end of the flexible outer shaft.

### Example 13

The endoscope assembly of any one or more of Examples 1 through 12, wherein the inflatable member defines an open distal end in the inflated configuration.

### Example 14

The endoscope assembly of any one or more of Examples 1 through 13, wherein the flexible outer shaft is resilient.

### Example 15

An endoscope assembly, comprising: (a) a shaft assembly, wherein the shaft assembly comprises: (i) a flexible outer shaft defining a lumen, wherein the flexible outer shaft comprises a distal end, (ii) an electrical communication line, and (iii) a fluid communication line; (b) an endoscopic camera assembly associated with the shaft assembly, wherein the endoscopic camera assembly is in communication with the electrical communication line; and (c) an anatomy elevation assembly comprising an inflatable member configured to transition between a deflated configuration and an inflated configuration, wherein the fluid communication line is in fluid communication with the inflatable member, wherein the inflatable member comprises a proximal end and a distal end, wherein the proximal end is coupled to the flexible outer shaft, wherein the inflatable member comprises an interior surface extending distally past the distal end of the flexible outer shaft, wherein an interior of the inflatable member is configured to define a viewing path distal to the endoscopic camera assembly while the inflatable member is in the inflated configuration.

### Example 16

The endoscope assembly of Example 15, wherein the distal end of the flexible outer shaft is open.

### Example 17

The endoscope assembly of Example 16, wherein the endoscopic camera assembly is housed within the lumen.

### Example 18

The endoscope assembly of any one or more of Examples 15 through 17, wherein the shaft assembly further comprises a working catheter housed within the lumen.

### Example 19

The endoscopic assembly of any one or more of Examples 15 through 18, wherein the endoscopic camera assembly comprises a camera portion and a light source portion.

### Example 20

An endoscope assembly, comprising: (a) a shaft assembly, wherein the shaft assembly comprises: (i) a flexible outer shaft defining a lumen, (ii) a first electrical communication line, and (iii) a first fluid communication line; (b) an endoscopic camera assembly associated with the shaft assembly, wherein the endoscopic camera assembly is in communication with the first electrical communication line; and (c) an anatomy elevation assembly comprising an inflatable member, wherein the first fluid communication line is in fluid communication with the inflatable member, wherein the inflatable member is configured to transition between a deflated configuration and an inflated configuration, wherein the inflatable member is configured to defines a viewing path distal to the endoscopic camera assembly in the inflated configuration, wherein the viewing path is unobstructed by the inflatable member.

### Example 21

The endoscope assembly of Example 6, further comprising a working element slidably disposed in the working catheter, the working element comprising one or more of a forceps, a swab, or a spray tube.

### V. Miscellaneous

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, versions of the device may be disassembled, and any number of the particular pieces or parts of the device may be selectively replaced or removed in any combination. Upon cleaning and/or replacement of particular parts, versions of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a surgical facility. A device may also be sterilized using any other technique known in the art, including but not limited to beta or gamma radiation, ethylene oxide, or steam.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An endoscope assembly, comprising:
(a) a shaft assembly, wherein the shaft assembly comprises:
(i) a flexible outer shaft defining a lumen,
(ii) a first electrical communication line, and
(iii) a first fluid communication line;
(b) an endoscopic camera assembly associated with the shaft assembly, wherein the endoscopic camera assembly is in communication with the first electrical communication line; and
(c) an anatomy elevation assembly comprising an inflatable member coupled to the flexible outer shaft, wherein the inflatable member comprises an interior surface, wherein the first fluid communication line is in fluid communication with the inflatable member, wherein the inflatable member is configured to transition between a deflated configuration and an inflated configuration, wherein the interior surface is configured to define a viewing path distal to the endoscopic camera assembly while the inflatable member is in the inflated configuration.

2. The endoscope assembly of claim 1, wherein the fluid communication line extends at least partially within the lumen, and /or the electrical communication line extends at least partially within the lumen.

3. The endoscope assembly of claim 1 or claim 2, wherein the interior surface is configured to define a frusto-conical fashion while the inflatable member is in the inflated configuration, or the interior surface is configured to extend in an arched fashion while the inflatable member is in the inflated configuration.

4. The endoscope assembly of any proceeding claim, wherein the shaft assembly further comprises a working catheter extending within the lumen of the flexible outer shaft, wherein the working catheter is dimensioned to slidably receive a working element.

5. The endoscope assembly of claim 4, further comprising a working element slidably disposed in the working catheter, the working element comprising one or more of a forceps, a swab, or a spray tube.

6. The endoscope assembly of any preceding claim, wherein the inflatable member comprises a proximal portion and a distal portion, wherein the proximal portion is attached to the flexible outer shaft, wherein the distal portion includes the interior surface.

7. The endoscope assembly of any preceding claim, further comprising a second electrical communication line in communication with a sensor element housed within the lumen, wherein the sensor element is operable to generate a position-indicative signal in response to an alternating magnetic field.

8. The endoscope assembly of claim 6 or claim 7, wherein the sensor element comprises a coil.

9. The endoscope assembly of any preceding claim, further comprising a second fluid communication line in communication with a fluid port configured to spray fluid on the camera assembly.

10. The endoscope assembly of any preceding claim, further comprising an actuator attached to a proximal end of the flexible outer shaft.

11. The endoscope assembly of any preceding claim, wherein the inflatable member defines an open distal end in the inflated configuration.

12. The endoscope assembly of any preceding claim, wherein the flexible outer shaft is resilient.

13. An endoscope assembly, comprising:
(a) a shaft assembly, wherein the shaft assembly comprises:
(i) a flexible outer shaft defining a lumen, wherein the flexible outer shaft comprises a distal end,
(ii) an electrical communication line, and
(iii) a fluid communication line;
(b) an endoscopic camera assembly associated with the shaft assembly, wherein the endoscopic camera assembly is in communication with the electrical communication line; and
(c) an anatomy elevation assembly comprising an inflatable member configured to transition between a deflated configuration and an inflated configuration, wherein the fluid communication line is in fluid communication with the inflatable member, wherein the inflatable member comprises a proximal end and a distal end, wherein the proximal end is coupled to the flexible outer shaft, wherein the inflatable member comprises an interior surface extending distally past the distal end of the flexible outer shaft, wherein an interior of the inflatable member is configured to define a viewing path distal to the endoscopic camera assembly while the inflatable member is in the inflated configuration.

14. An endoscope assembly, comprising:
(a) a shaft assembly, wherein the shaft assembly comprises:
(i) a flexible outer shaft defining a lumen,
(ii) a first electrical communication line, and
(iii) a first fluid communication line;
(b) an endoscopic camera assembly associated with the shaft assembly, wherein the endoscopic camera assembly is in communication with the first electrical communication line; and
(c) an anatomy elevation assembly comprising an inflatable member, wherein the first fluid communication line is in fluid communication with the inflatable member, wherein the inflatable member is configured to transition between a deflated configuration and an inflated configuration, wherein the inflatable member is configured to defines a viewing path distal to the endoscopic camera assembly in the inflated configuration, wherein the viewing path is unobstructed by the inflatable member.
